# EUROPEAN PATENT APPLICATION

(11) **EP 3 563 829 A1**
(43) Date of publication of application: **06.11.2019**
(21) Application number: 17886675.2
(22) Date of filing: 28.02.2017
(51) Int. Cl.: A61K 8/64, A61K 8/04, A61K 8/02, A61Q 19/08, A61P 17/02, A61P 29/00

(54) **COSMETIC COMPOSITION**

(30) Priority: 29.12.2016 CN 201611243092
(71) Applicant: Shaanxi Huikang Bio-Tech Co., Ltd, Xi'an, Shaanxi 710054 (CN)
(72) Inventor: HOU, Zengmiao, Xi'an Shaanxi 710054 (CN); LONG, Fei, Xi'an Shaanxi 710054 (CN); YANG, Xiaolin, Xi'an Shaanxi 710054 (CN); ZHAO, Jinli, Xi'an Shaanxi 710054 (CN)
(74) Representative: Habermann, Hruschka & Schnabel
(86) International application number: PCT/CN2017/075126
(87) International publication number: WO 2018/120401

(57) **Abstract**

A cosmetic composition, comprising osteogenic growth peptide carbon-terminal pentapeptide, and at least one of a carrier, a diluent and an adjuvant. The cosmetic composition can promote the proliferation of fibroblasts and the synthesis of skin collagen, and also has the effects of promoting skin wound healing, and eliminating swelling and inflammation.

## Description

### Technical Field

The present invention belongs to the technical field of cosmetics, and in particular, the present invention relates to a cosmetic composition comprising C-terminal pentapeptide of osteogenic growth peptide.

### Background of the Invention

Osteogenic Growth Peptide (known as OGP for short) is a polypeptide which is discovered in humans and animals by I.Bab et al. (The Hebrew University of Jerusalem, Israel) in 1988 and isolated from a regenerated bone marrow cell in 1992. In vitro experiments have shown that the polypeptide may stimulate proliferate and differentiate of osteoblastic cells and bone marrow mesenchymal stem cells, promote osteogenesis, and increase synthesis of collagen type I, therefore, it is named OGP. In vivo experiments have shown that OGP may activate hematopoietic system, and promote bone healing.

OGP is commonly found in mammalian serum, and the sequences thereof possess high conservatism. OGP has 14 amino acids in length, and has a molecular weight of 1523.8 Da. The sequence of OGP is Ala-Leu-Lys-Arg-Gln-Gly-Arg-Thr-Leu-Tyr-Gly-Phe-Gly-Gly, which is identical to the C-terminal residues 89 to 102 of histone H4. Due to effects of protease *in vivo,* unbound OGP is easily degraded by protease into C-terminal pentapeptide of osteogenic growth peptide Tyr-Gly-Phe-Gly-Gly (OGP (10-14)). OGP (10-14) is a minimal active sequence of OGP, which retains mitogenic activity and *in vivo* effects of OGP, of which Tyr10 and Phe12 side chains as well as terminal Gly residues are important for optimal bioactivity of OGP.

In addition to stimulating proliferation of osteoblastic cells, OGP and OGP (10-14) can also stimulate proliferation fibroblastic cells. In vitro experiments have shown that NIH3T3 fibroblastic cells may have peak proliferation when exogenous OGP has a concentration of 10⁻¹¹ to 10⁻¹⁰ mol/L.

There is currently no application of osteogenic growth peptide and C-terminal pentapeptide of osteogenic growth peptide in the preparation of cosmetics. There is no literature to report that osteogenic growth peptide and C-terminal pentapeptide of osteogenic growth peptide have functions of anti-inflammatory and reducing swelling. Based on studies on C-terminal pentapeptide of osteogenic growth peptide, it is applied to cosmetics in present invention. The cosmetic composition has effects of wrinkle removal and anti-aging, while it has effects of anti-inflammatory and eliminating redness caused by minimal invasion.

### Summary of the Invention

The objective of the present invention is to provide a new use of C-terminal pentapeptide of osteogenic growth peptide to overcome the defects existed in prior art, i.e. the use of the C-terminal pentapeptide in the preparation of a cosmetic composition. In particular, the present invention is achieved by the following technical solutions:
There is provided a cosmetic composition in the present invention, which comprises C-terminal pentapeptide of osteogenic growth peptide, as well as at least one of a carrier, a diluent and an adjuvant.

In the aforementioned cosmetic composition, the C-terminal pentapeptide of osteogenic growth peptide is added in an amount of 0.5 µg/ml to 10 µg/ml, preferably 5 µg/ml, based on the volume of the cosmetic composition.

In the aforementioned cosmetic composition, the C-terminal pentapeptide of osteogenic growth peptide has an amino acid sequence of Tyr-Gly-Phe-Gly-Gly, a molecular weight of 499.5 Daltons, and an isoelectric point of 5.52.

In the aforementioned cosmetic composition, the cosmetic composition is in the form of a toning lotion, an emulsion, a cream, a gel or a facial mask.

In the aforementioned cosmetic composition, the cosmetic composition is a toning lotion comprising a lyophilized powder and a menstruum, with the volume ratio of the lyophilized powder to the menstruum of 1 : (2-5);
wherein the lyophilized powder is prepared by lyophilizing a mixture of mannitol, C-terminal pentapeptide of osteogenic growth peptide and sodium phosphate buffer, wherein each milliliter of the mixture comprises 10 mg to 50 mg of mannitol, 0.5 µ g to 10 µ g of C-terminal pentapeptide of osteogenic growth peptide, and the balance of sodium phosphate buffer;
wherein each milliliter of the menstruum comprises 0.5 mg to 2 mg of low molecular weight sodium hyaluronate, 0.1 mg to 1 mg of sodium hyaluronate, 10 mg to 80 mg of sodium lactate, 10 mg to 80 mg of glycerol, 0.5 mg to 5 mg of dipotassium glycyrrhizinate, and the balance of sodium phosphate buffer.

In the aforementioned cosmetic composition, the cosmetic composition is a toning lotion comprising a lyophilized powder and a menstruum, with the volume ratio of the lyophilized powder to the menstruum of 1 : 2;
wherein the lyophilized powder is prepared by lyophilizing a mixture of mannitol, C-terminal pentapeptide of osteogenic growth peptide and sodium phosphate buffer, wherein each milliliter of the mixture comprises 30 mg of mannitol, 10 µg of C-terminal pentapeptide of osteogenic growth peptide, and the balance of sodium phosphate buffer;
wherein each milliliter of the menstruum comprises 0.8 mg of low molecular weight sodium hyaluronate, 0.2 mg of sodium hyaluronate, 60 mg of sodium lactate, 40 mg of glycerol, 3 mg of dipotassium glycyrrhizinate, and the balance of sodium phosphate buffer.

In the aforementioned cosmetic composition, the cosmetic composition is a gel; wherein each liter of the gel comprises 0.5 mg to 10 mg of C-terminal pentapeptide of osteogenic growth peptide, 2 g to 8 g carbomer, 2 g to 10 g sodium hyaluronate, 10 g to 50 g glycerol, 3 ml to 10 ml phenoxyethanol, and the balance of deionized water.

In the aforementioned cosmetic composition, the cosmetic composition is a gel; wherein each liter of the gel comprises 5 mg of C-terminal pentapeptide of osteogenic growth peptide, 5 g carbomer, 4 g sodium hyaluronate, 30 g glycerol, 7 ml phenoxyethanol, and the balance of deionized water.

Compared with prior art, the cosmetic composition provided by the present invention at least has the following advantages: the cosmetic composition according to the present invention not only can promote proliferation of fibroblastic cells, stimulate synthesis of collagen, promote regeneration of skin cells, and promote healing of skin micro-wound, but also can accelerate elimination of skin redness and inflammation by minimal invasion, and reduce discomfort after minimally invasive surgery.

### Brief Description of the Drawings

Figure 1 illustrates a result of the cell proliferation test with the cosmetic composition of the present invention.
Figure 2 illustrates the use effect of the cosmetic composition of the present invention after minimally invasive surgery of seaweed silicon needle.

### Description of Embodiments

In order to fully understand the purposes, characteristics and effects of the present invention, the present invention will be described in detail with reference to the following detailed description.

The cosmetic composition according to the present invention comprises C-terminal pentapeptide of osteogenic growth peptide, as well as at least one selected from the group consisting of a carrier, a diluent and an adjuvant. C-terminal pentapeptide of osteogenic growth peptide is a minimal active sequence of osteogenic growth peptide, which retains mitogenic activity and *in vivo* effects of osteogenic growth peptide. The C-terminal pentapeptide of osteogenic growth peptide has an amino acid sequence of Tyr-Gly-Phe-Gly-Gly, a molecular weight of 499.5 Daltons, and an isoelectric point of 5.52. The C-terminal pentapeptide of osteogenic growth peptide used in the present invention is prepared with the method of Chinese application for invention No. 201310578687.2, the entire content of which is herein incorporated by reference.

The cosmetic composition according to the present invention comprises carriers, adjuvants and additives commonly used in preparation of cosmetics, such as water, mask stickers, excipients, humectants, thickeners, preservatives, colorants, emulsifiers, stabilizers, perfumes, and the like.

The C-terminal pentapeptide of osteogenic growth peptide is added in an amount of 0.5 µg/ml to 10 µg/ml, and preferably 5 µg/ml, based on the volume of the cosmetic composition.

The cosmetic composition according to the present invention may be presented in any form, such as, a toning lotion, an emulsion, a cream, a gel, a facial mask or the like. Those skilled in the art will select a conventional preparation method according to the particular form of the cosmetic composition.

In a prefered embodiment, the cosmetic composition according to the present application is a toning lotion comprising:
(1) a lyophilized powder, which is prepared by lyophilizing a mixture of mannitol, C-terminal pentapeptide of osteogenic growth peptide and sodium phosphate buffer; wherein each milliliter of the mixture comprises 10 mg to 50 mg of mannitol, 0.5 µg to 10 µg of C-terminal pentapeptide of osteogenic growth peptide, and the balance of sodium phosphate buffer; and
(2) a menstruum, wherein each milliliter of the menstruum comprises 0.5 mg to 2 mg of low molecular weight sodium hyaluronate, 0.1 mg to 1 mg of sodium hyaluronate, 10 mg to 80 mg of sodium lactate, 10 mg to 80 mg of glycerol, 0.5 mg to 5 mg of dipotassium glycyrrhizinate, and the balance of sodium phosphate buffer.

The volume ratio of the lyophilized powder to the menstruum is 1 : (2-5).

Wherein, sodium phosphate buffer can be used in the concentrations and pH values commonly used in the art, as long as it can protect the activities of C-terminal pentapeptide of osteogenic growth peptide. For example, sodium phosphate buffer may have a concentration of 5 mM and a pH value of 6.5.

The sodium hyaluronate used in the present invention has a molecular weight of 1,200,000 to 1,500,000 Daltons, and the low molecular weight sodium hyaluronate has a molecular weight of 200,000 to 400,000 Daltons.

The substances involved in the above-mentioned toning lotion may be commercially available or prepared by conventional methods.

The above-mentioned toning lotion employs a lyophilized powder and a menstruum, and the menstruum functions as a dissolving agent. In use, the menstruum dissolves the lyophilized powder in order to ensure the maximum extent activities of C-terminal pentapeptide of osteogenic growth peptide in the toning lotion. At the same time, with the specific composition and the specific ratio, there is a synergistic effect among the components of the menstruum, which is more conducive to disperse and dissolve the lyophilized powder, such that the skin absorption of nutrients can be promoted in use, and activities of C-terminal pentapeptide of osteogenic growth peptide can be maintained for a prolonged period of time.

In a particularly preferred embodiment, the toning lotion comprises a lyophilized powder and a menstruum in a volume ratio of the lyophilized powder to the menstruum of 1 : 2; wherein the lyophilized powder is prepared by lyophilizing a mixture of mannitol, C-terminal pentapeptide of osteogenic growth peptide and sodium phosphate buffer, wherein each milliliter of the mixture comprises 30 mg of mannitol, 10 µg of C-terminal pentapeptide of osteogenic growth peptide, and the balance of sodium phosphate buffer; wherein each milliliter of the menstruum comprises 0.8 mg of low molecular weight sodium hyaluronate, 0.2 mg of sodium hyaluronate, 60 mg of sodium lactate, 40 mg of glycerol, 3 mg of dipotassium glycyrrhizinate, and the balance of sodium phosphate buffer.

In another particularly preferred embodiment, the cosmetic composition is a gel; wherein each liter of the gel comprises 0.5 mg to 10 mg of C-terminal pentapeptide of osteogenic growth peptide, 2 g to 8 g carbomer, 2 g to 10 g sodium hyaluronate, 10 g to 50 g glycerol, 3 ml to 10 ml phenoxyethanol, and the balance of deionized water.

The sodium hyaluronate has a molecular weight of 1,200,000 to 1,500,000 Daltons.

The above substances involved may be commercially available.

With the specific composition and the specific ratio, there is a synergistic effect among the components of the gel, which is more conducive to disperse and dissolve the lyophilized powder, such that the skin absorption of nutrients can be promoted in use, and activities of C-terminal pentapeptide of osteogenic growth peptide can be maintained for a prolonged period of time.

In another particularly preferred embodiment, each liter of the gel comprises 5 mg of C-terminal pentapeptide of osteogenic growth peptide, 5 g carbomer, 4 g sodium hyaluronate, 30 g glycerol, 7 ml phenoxyethanol, and the balance of deionized water.

### Examples

The present invention is further illustrated by way of examples, but the invention is not limited to the scope of the described embodiments. The experimental methods which are not specified in the following examples are performed according to conventional methods and conditions, or according to product specifications. The substances used in the examples are either commercially available or prepared by conventional methods.

### Example 1: Toning lotion comprising C-terminal pentapeptide of osteogenic growth peptide and preparation of the same

The cosmetic composition comprising C-terminal pentapeptide of osteogenic growth peptide in this example is a toning lotion comprising a lyophilized powder and a menstruum. Each lyophilized powder has a volume of 1 ml, each menstruum has a volume of 2 ml, and each lyophilized powder is used with each menstruum. Wherein each lyophilized powder comprises 30 mg of mannitol, 10 µg of C-terminal pentapeptide of osteogenic growth peptide, and the balance of 5 mM, pH 6.5 sodium phosphate buffer; wherein each menstruum comprises 1.6 mg of low molecular weight sodium hyaluronate, 0.4 mg of sodium hyaluronate, 120 mg of sodium lactate, 80 mg of glycerol, 6 mg of dipotassium glycyrrhizinate, and the balance of 5 mM, pH 6.5 sodium phosphate buffer.

The above-mentioned toning lotion is prepared by the following steps:
(1) 30 mg of mannitol and 10 µg of C-terminal pentapeptide of osteogenic growth peptide were dissolved in 5 mM of sodium phosphate buffer with a pH of 6.5, diluted to volume of 1 ml, subjected to sterile filtration, and lyophilized by lyophilizer to obtain the lyophilized powder;
(2) 1.6 mg of low molecular weight sodium hyaluronate, 0.4 mg of sodium hyaluronate, 120 mg of sodium lactate, 80 mg of glycerol, and 6 mg of dipotassium glycyrrhizinate were successively added into 5 mM of sodium phosphate buffer with a pH of 6.5, the resulting mixture were diluted with 5 mM of sodium phosphate buffer with a pH of 6.5 to a volume of 2 ml, and autoclaved at 120 ° C and high pressure for 30 minutes so as to obtain the menstruum.

Before use, the lyophilized powder is dissolved into the menstruum. One lyophilized powder is dissolved with one menstruum.

### Example 2: Gel comprising C-terminal pentapeptide of osteogenic growth peptide and preparation of the same

The cosmetic composition comprising C-terminal pentapeptide of osteogenic growth peptide in this example is a gel comprising C-terminal pentapeptide of osteogenic growth peptide, carbomer, sodium hyaluronate, glycerol, phenoxyethanol, and deionized water; wherein each liter of the gel comprises 5 mg of C-terminal pentapeptide of osteogenic growth peptide, 5 g carbomer, 4 g sodium hyaluronate, 30 g glycerol, 7 ml phenoxyethanol, and the balance of deionized water.

The above-mentioned gel is prepared by the following steps:
4 g of sodium hyaluronate was slowly added into 400 ml of deionized water, stirred and dissolved; then the resulting solution was added with 5 mg of C-terminal pentapeptide of osteogenic growth peptide, 30 g of glycerin, 7 ml of phenoxyethanol, respectively, and stirred uniformly to obtain phase A; 5 g carbomer was uniformly spread into 400 ml of water, and stirred to completely swell so as to obtain phase B; then phase A was add into phase B, the resulting mixture was adjusted to pH of 6.5 with NaOH, diluted with deionized water to volume, and stirred uniformly to give the finished product.

### Example 3: Proliferation test of cosmetic composition comprising C-terminal pentapeptide of osteogenic growth peptide

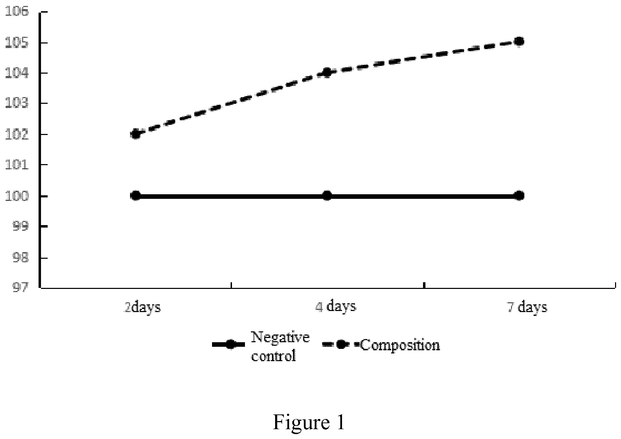

A single cell suspension with a concentration of 6 × 10³ cells/ml was obtained by diluting fibroblastic cells separated from cultured human skin with DMEM/F12 culture solution containing 10% fetal bovine serum. Three 96-well plates were used, and 40 wells of each plates were inoculated with 100 µl of the suspension. The three 96-well plates were placed into an incubator containing 5 vt.% CO₂ to incubate for 24 hours at 37 °C. The original culture solution was discarded. Each well was added with 100 µl of the cosmetic composition comprising C-terminal pentapeptide of osteogenic growth peptide prepared in Example 1 or a negative control solution (only cell culture solution), and each group comprises 8 wells. The three plates were transferred to an incubator at 37 °C which contains 5 vt.% CO₂. The three plates were respectively taken on days 2, 4, and 7 after inoculation, each well of which was added with 50 µl of MTT (thiazolyl blue), and were incubated at 37 °C for another 2 hours. The original culture solution was discarded, and 150 µl of dimethyl sulfoxide was immediately added into each well. The three plates were placed at room temperature and gently shook for 10 to 15 min. Absorbance (A) of each well was measured with a microplate reader at 490 nm, and the relative proliferation rates of cells RGR (%) were calculated according to the following formula: RGR (%) = (absorbance of experimental group / absorbance of negative control group) ×100%. As can be seen from the results in Figure 1, the relative proliferation rate of the cosmetic composition comprising C-terminal pentapeptide of osteogenic growth peptide increases with the increase in culture days.

### Example 4: Skin safety test and anti-aging test of cosmetic composition comprising C-terminal pentapeptide of osteogenic growth peptide

There were 20 social volunteers aged 22 to 65 years old, including 8 males and 12 females. The cosmetic composition comprising C-terminal pentapeptide of osteogenic growth peptide prepared in Example 1 was applied to the same side face and back of the hand twice a day (the concentration of C-terminal pentapeptide of osteogenic growth peptide was 5 µl/ml, and the dosage was 1 ml), for 14 days continuously. The results were shown as follow: every subject had no itching, redness or skin ulceration. Taken the unapplied side face and back of the hand as controls, 85% of the tested skins were more moist, smooth and shiny, and wrinkles at corners of mouth and eyes were significantly reduced.

### Example 5: Anti-inflammatory and swelling reduction efficacy test of cosmetic composition comprising C-terminal pentapeptide of osteogenic growth peptide

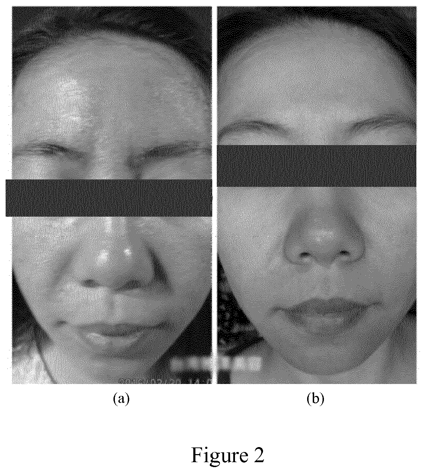

There were 17 social volunteers aged 20 to 50 years old, including 4 males and 6 females in an experimental group, and 3 males and 4 females in a control group. After treated with seaweed silicon needle, facial skins of the members of the experimental group were applied with the cosmetic composition comprising C-terminal pentapeptide of osteogenic growth peptide prepared in Example 1 once (the concentration of C-terminal pentapeptide of osteogenic growth peptide was 5 µl/ml, and the dosage is 1 ml). Facial skins of the members of the control group were only treated with seaweed silicon needle. The results were shown in Figure 2. In Figure 2, (a) showed a redness state after treatment with seaweed silicon needle; and (b) showed a skin state 24 hours after the facial skin which has been treated with seaweed silicon needle was applied with the cosmetic composition comprising C-terminal pentapeptide of osteogenic growth peptide prepared in Example 1. It could be seen from Figure 2 that the redness and tingling of facial skin of every tested subject disappeared within 24 hours, while the redness and tingling of facial skin of every member in the control group lasted for more than 5 days.

Finally, it is to be noted that the foregoing is merely illustrative of specific embodiments of the present invention, and that modifications and variations of the present invention may be made by those skilled in the art. If such modifications and variations are within the scope of the appended claims and their equivalents, they shall be deemed to be within the scope of protection of the present invention.

## Claims

1. A cosmetic composition, **characterized in that**, the cosmetic composition comprises C-terminal pentapeptide of osteogenic growth peptide, as well as at least one of a carrier, a diluent and an adjuvant.

2. The cosmetic composition of claim 1, **characterized in that**, the C-terminal pentapeptide of osteogenic growth peptide is added in an amount of 0.5 µg/ml to 10 µg/ml, preferably 5 µg/ml, based on the volume of the cosmetic composition.

3. The cosmetic composition of claim 1 or 2, **characterized in that**, the C-terminal pentapeptide of osteogenic growth peptide has an amino acid sequence of Tyr-Gly-Phe-Gly-Gly, a molecular weight of 499.5 Daltons, and an isoelectric point of 5.52.

4. The cosmetic composition of any one of claims 1 to 3, **characterized in that**, the cosmetic composition is a toning lotion, an emulsion, a cream, a gel or a facial mask.

5. The cosmetic composition of any one of claims 1 to 4, **characterized in that**, the cosmetic composition is a toning lotion comprising a lyophilized powder and a menstruum, with the volume ratio of the lyophilized powder to the menstruum of 1 : 2 to 5;
wherein the lyophilized powder is prepared by lyophilizing a mixture of mannitol, C-terminal pentapeptide of osteogenic growth peptide and sodium phosphate buffer,
wherein each milliliter of the mixture comprises 10 mg to 50 mg of mannitol, 0.5 µg to 10 µg of C-terminal pentapeptide of osteogenic growth peptide, and the balance of sodium phosphate buffer;
wherein each milliliter of the menstruum comprises 0.5 mg to 2 mg of low molecular weight sodium hyaluronate, 0.1 mg to 1 mg of sodium hyaluronate, 10 mg to 80 mg of sodium lactate, 10 mg to 80 mg of glycerol, 0.5 mg to 5 mg of dipotassium glycyrrhizinate, and the balance of sodium phosphate buffer.

6. The cosmetic composition of any one of claims 1 to 4, **characterized in that**, the cosmetic composition is a toning lotion comprising a lyophilized powder and a menstruum, with the volume ratio of the lyophilized powder to the menstruum of 1 : 2;
wherein the lyophilized powder is prepared by lyophilizing a mixture of mannitol, C-terminal pentapeptide of osteogenic growth peptide and sodium phosphate buffer,
wherein each milliliter of the mixture comprises 30 mg of mannitol, 10 µg of C-terminal pentapeptide of osteogenic growth peptide, and the balance of sodium phosphate buffer;
wherein each milliliter of the menstruum comprises 0.8 mg of low molecular weight sodium hyaluronate, 0.2 mg of sodium hyaluronate, 60 mg of sodium lactate, 40 mg of glycerol, 3 mg of dipotassium glycyrrhizinate, and the balance of sodium phosphate buffer.

7. The cosmetic composition of any one of claims 1 to 4, **characterized in that**, the cosmetic composition is a gel; wherein each liter of the gel comprises 0.5 mg to 10 mg of C-terminal pentapeptide of osteogenic growth peptide, 2 g to 8 g carbomer, 2 g to 10 g sodium hyaluronate, 10 g to 50 g glycerol, 3 ml to 10 ml phenoxyethanol, and the balance of deionized water.

8. The cosmetic composition of any one of claims 1 to 4, **characterized in that**, the cosmetic composition is a gel; wherein each liter of the gel comprises 5 mg of C-terminal pentapeptide of osteogenic growth peptide, 5 g carbomer, 4 g sodium hyaluronate, 30 g glycerol, 7 ml phenoxyethanol, and the balance of deionized water.
